# EUROPEAN PATENT APPLICATION

(11) **EP 4 524 990 A1**
(43) Date of publication of application: **19.03.2025**
(21) Application number: 24196862.7
(22) Date of filing: 28.08.2024
(51) Int. Cl.: G16H 50/20, G16H 50/30, G16H 50/50, G16H 50/70

(54) **MULTI-LABEL CLASSIFICATION METHOD AND DEVICE THAT MEET DEPRESSIVE DISORDER DIAGNOSTIC CRITERIA**

(30) Priority: 30.08.2023 KR 20230114709
(71) Applicant: Samsung Life Public Welfare Foundation, Seoul 04348 (KR); Research & Business Foundation Sungkyunkwan University, Jangan-gu Suwon-si, Gyeonggi-do 16419 (KR)
(72) Inventor: OH, Ha Young, 06567 Seoul (KR); KIM, Seog Ju, 06351 Seoul (KR); PARK, Da Bin, 17863 Gyeonggi-do (KR); LIM, Se Min, 12798 Gyeonggi-do (KR); CHOI, Yu Rim, 02748 Seoul (KR)
(74) Representative: Weickmann & Weickmann PartmbB

(57) **Abstract**

A training method according to an embodiment may include: performing transfer learning a first artificial neural network model based on a consultation dataset sentence; inputting depressive disorder-related expression data into the first artificial neural network model, labeling the depressive disorder-related expression data according to depressive disorder diagnosis criteria, and generating labeled depressive disorder-related expression data; and training a second artificial neural network model based on the labeled depressive disorder-related expression data so that a second artificial neural network model may output the depressive disorder diagnosis criteria corresponding to input data.

## Description

### BACKGROUND

### 1. Field

One or more embodiments relate to a multi-label classification method and device that meet depressive disorder diagnostic criteria.

### 2. Description of the Related Art

The prevalence of depressive disorder in Korea is the highest among OECD countries. The rate of depressive disorder among young people in their 20s is very high, and because depressive disorder causes a decline in daily functioning, it is important to detect it quickly and treat it appropriately.

The DSM-5, the most recent version of the Diagnostic and Statistical Manual of Mental Disorders published by the American Psychiatric Association, is used in many countries. The Korean psychiatric society also uses DSM-5 diagnostic criteria to diagnose depressive disorder.

Currently, research is actively being conducted to detect depressive disorder, but there is no dataset labeled based on the DSM-5 diagnostic criteria. Therefore, there is no research predicting depressive disorder based on the DSM-5 diagnostic criteria.

### SUMMARY

A training method according to an embodiment may include: performing transfer learning a first artificial neural network model based on a consultation dataset sentence; inputting depressive disorder-related expression data into the first artificial neural network model, labeling the depressive disorder-related expression data according to depressive disorder diagnosis criteria, and generating labeled depressive disorder-related expression data; and training a second artificial neural network model based on the labeled depressive disorder-related expression data so that a second artificial neural network model may output the depressive disorder diagnosis criteria corresponding to input data.

The generating of labeled depressive disorder-related expression data may include: extracting a plurality of depressive disorder diagnostic criteria corresponding to the depressive disorder-related expression data from the depressive disorder diagnostic criteria; and multi-labeling the depressive disorder-related expression data based on the plurality of depressive disorder diagnostic criteria.

The generating of labeled depressive disorder-related expression data may include: obtaining first output data by inputting the depressive disorder-related expression data into the first artificial neural network model; converting the first output data into second output data based on a sigmoid function, the second output data comprising a plurality of components corresponding to the depressive disorder diagnostic criteria, respectively; and labeling the depressive disorder-related expression data based on the depressive disorder diagnostic criteria corresponding to a component equal to or greater than a preset threshold value from among the plurality of components.

The depressive disorder diagnostic criteria may include DMS-5 depressive disorder diagnostic criteria.

The training may include: training the second artificial neural network model so that the second artificial neural network model may output the depressive disorder diagnosis criteria corresponding to the input data and probability corresponding to the depressive disorder diagnosis criteria.

The generating of labeled depressive disorder-related expression data may include: performing data augmentation on residual data from among the depressive disorder-related expression data that is not labeled by the first artificial neural network model; and re-inputting residual data expanded by the data augmentation into the first artificial neural network to label the expanded residual data according to the depressive disorder diagnosis criteria.

The first artificial neural network model may include a KoBERT model, and the second artificial neural network model may include a GRU model.

A training device according to an embodiment may include: a first artificial neural network model that performs transfer learning on sentences from a consultation dataset, labels depressive disorder-related expression data according to depressive disorder diagnosis criteria, and generates labeled depressive disorder-related expression data; and a second artificial neural network model trained to output the depressive disorder diagnosis criteria corresponding to the labeled depressive disorder-related expression data.

The first artificial neural network model may be configured to: extract a plurality of depressive disorder diagnostic criteria corresponding to the depressive disorder-related expression data from the depressive disorder diagnostic criteria; and multi-label the depressive disorder-related expression data based on the plurality of depressive disorder diagnosis criteria to generate the labeled depressive disorder-related expression data.

The first artificial neural network model may be configured to: obtain first output data by inputting depressive disorder-related expression data into the first artificial neural network model; convert the first output data into second output data including a plurality of components corresponding to the depressive disorder diagnosis criteria, respectively, based on a sigmoid function; and label the depressive disorder-related expression data based on the depressive disorder diagnostic criteria corresponding to a component equal to or greater than a preset threshold value from among the plurality of components to generate the labeled depressive disorder-related expression data.

The depressive disorder diagnostic criteria may include DMS-5 depressive disorder diagnostic criteria.

The second artificial neural network model may be trained to output the depressive disorder diagnosis criteria corresponding to the labeled depressive disorder-related expression data and probability corresponding to the depressive disorder diagnosis criteria.

The first artificial neural network model may be configured to: perform data augmentation on unlabeled residual data from among the depressive disorder-related expression data; and re-input expanded residual data through the data augmentation, label the expanded residual data according to the depressive disorder diagnosis criteria, and generate the labeled depressive disorder-related expression data.

The first artificial neural network model may include a KoBERT model, and the second artificial neural network model may include a GRU model.

### BRIEF DESCRIPTION OF DRAWINGS

FIG. 1 is a schematic block diagram of a training device according to an embodiment.
FIG. 2 is a view for explaining DMS-5 depressive disorder diagnostic criteria.
FIG. 3 is a view schematically illustrating a training method according to an embodiment.
FIG. 4 is a view illustrating an example of a labeling result according to an embodiment.
FIGS. 5A and 5B are views for explaining multi-labeling.
FIG. 6 is a flowchart for explaining a training method according to an embodiment.

### DETAILED DESCRIPTION

Hereinafter, embodiments of the present invention will be described with reference to the drawings. In the following description, descriptions of a well-known technical configuration in relation to a lead implantation system for a deep brain stimulator will be omitted. For example, descriptions of the configuration/structure/method of a device or system commonly used in deep brain stimulation, such as the structure of an implantable pulse generator, a connection structure/method of the implantable pulse generator and a lead, and a process for transmitting and receiving electrical signals measured through the lead with an external device, will be omitted. Even if these descriptions are omitted, one of ordinary skill in the art will be able to easily understand the characteristic configuration of the present invention through the following description.

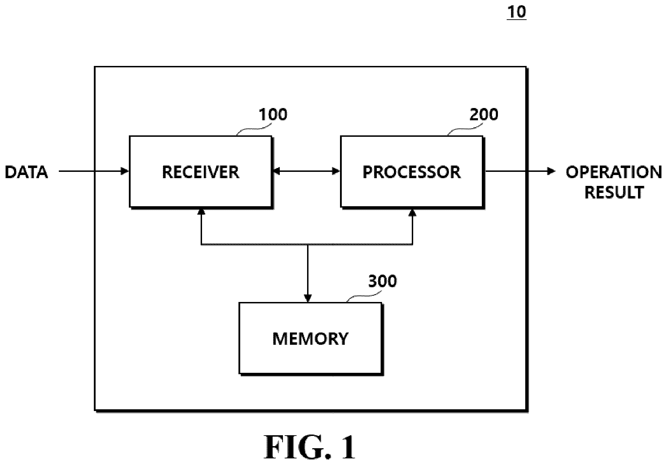

FIG. 1 is a schematic block diagram of a training device according to an embodiment.

Referring to FIG. 1, the training device 10 may train an optimal model for detecting depressive disorder by introducing depressive disorder diagnostic criteria. In more detail, a system according to an embodiment requires a first artificial neural network model for performing multi-labeling and a second artificial neural network model for predicting depressive disorder, and the second artificial neural network model may be trained using multi-labeled data obtained by the first artificial neural network.

In other words, the training device 10 may perform transfer learning the first artificial neural network based on a consultation dataset sentence, and may multi-label depressive disorder-related expression data using the transfer-learned first artificial neural network according to the depressive disorder diagnostic criteria (e.g., the DSM-5). The training device 10 may train the second artificial neural network model using the multi-labeled data obtained by the first artificial neural network.

The training device 10 includes a receiver 100 and a processor 200. The training device 10 may further include a memory 300.

The receiver 100 may include a receiving interface. The receiver 100 may receive the consultation dataset sentence and the depressive disorder-related expression data.

The processor 200 may process data stored in the memory 300. The processor 200 may execute computer-readable code (e.g., software) stored in the memory 300 and instructions generated by the processor 200.

The processor 200 may be a data processing device implemented in hardware having a circuit with a physical structure for performing desired operations. For example, desired operations may include code or instructions included in a program.

For example, a data processing device implemented in hardware may be a microprocessor, a central processing unit, a processor core, a multi-core processor, a multiprocessor, an application-specific integrated circuit (ASIC), and a field programmable gate array (FPGA).

The memory 300 may store instructions (or programs) executable by the processor 200. For example, the instructions may include instructions for executing operations of the processor 200 and/or operations of each component of the processor 200.

The memory 300 may be implemented as a volatile memory device or a nonvolatile memory device.

The volatile memory device may be implemented as dynamic random access memory (DRAM), static random access memory (SRAM), thyristor RAM (T-RAM), zero capacitor RAM (Z-RAM), or twin transistor RAM (TTRAM).

The nonvolatile memory device may be implemented as electrically erasable programmable read-only memory (EEPROM), a flash memory, magnetic RAM (MRAM), spin-transfer torque (STT)-MRAM, conductive bridging RAM (CBRAM), ferroelectric RAM (FeRAM), phase change RAM (PRAM), resistive RAM (RRAM), nanotube RRAM, polymer RAM (PoRAM), nano floating gate memory (NFGM), a holographic memory, a molecular electronic memory device, or an insulator resistance change memory.

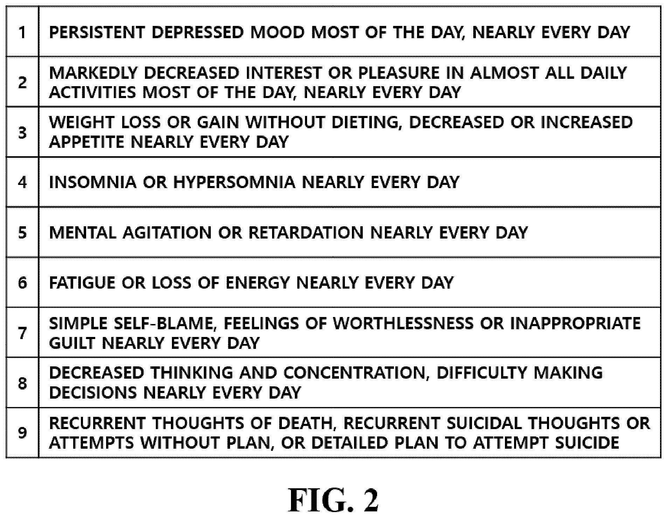

FIG. 2 is a view for explaining DMS-5 depressive disorder diagnostic criteria.

Referring to FIG. 2, as described above, a training device according to an embodiment may multi-label depressive disorder-related expression data according to depressive disorder diagnostic criteria (e.g., the DSM-5).

The Diagnostic and Statistical Manual of Mental Disorders 5th Edition (DSM-5) is a mental disorder diagnostic classification system officially used by the American Psychiatric Association (APA). The DSM-5 is used all over the world, and depressive disorder is also included in the classification of mental disorders. Depressive disorder is determined by comprehensively considering nine diagnostic criteria of the DSM-5, the clinical experience of psychiatrists, clinical interviews, psychological test results, and treatment progress.

In Korea, the DSM-5 is also used to diagnose depressive disorder, but the lack of specialized personnel including psychiatrists and the negative social perception of depressive disorder prevents counselees from treating depressive disorder.

Referring to FIG. 2, the DSM-5 has nine diagnostic criteria: 1) persistent depressed mood most of the day, nearly every day; 2) markedly decreased interest or pleasure in almost all daily activities most of the day, nearly every day; 3) weight loss or gain without dieting, decreased or increased appetite nearly every day; 4) insomnia or hypersomnia nearly every day; 5) mental agitation or retardation nearly every day; 6) fatigue or loss of energy nearly every day; 7) simple self-blame, feelings of worthlessness or inappropriate guilt nearly every day; 8) decreased thinking and concentration, difficulty making decisions nearly every day; and 9) recurrent thoughts of death, recurrent suicidal thoughts or attempts without a plan, or a detailed plan to attempt suicide.

A word dictionary according to an embodiment may be generated based on the DSM-5. In other words, the word dictionary may be composed of nine categories and words corresponding to the categories. For example, the category 3) 'weight loss or gain without dieting, decreased or increased appetite nearly every day' of the word dictionary may include words such as 'pig', 'fat', 'body weight', and 'appetite'. Furthermore, the words included in the word dictionary may be added according to a data augmentation algorithm.

In FIG. 2, depressive disorder diagnostic criteria are not limited to the DSM-5 described above, and labeling may be performed based on depressive disorder diagnostic criteria that are different from the DSM-5.

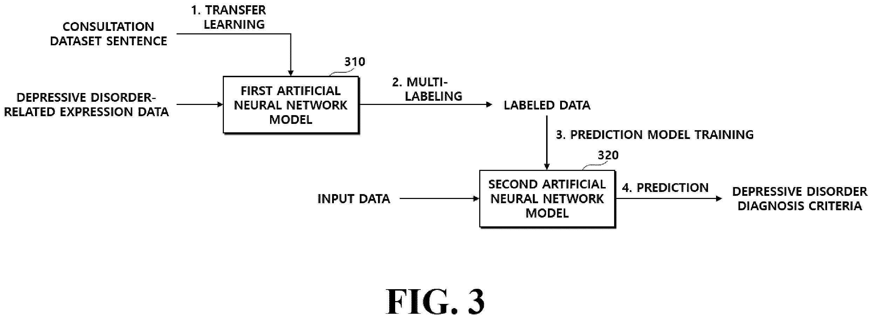

FIG. 3 is a view schematically illustrating a training method according to an embodiment.

The descriptions with reference to FIGS. 1 and 2 may be equally applied to FIG. 3.

Referring to FIG. 3, a training device according to an embodiment may perform transfer learning a first artificial neural network model 310 based on a consultation dataset sentence. The consultation dataset sentence may be, for example, a wellness conversation script dataset. The wellness conversation script dataset may be a user-chatbot conversation script generated by extracting cases (e.g., 4,200 cases) in which a patient visited the hospital for the first time and visited in person from among counseling data (e.g., 16,000 cases) received from the hospital, separating them by sentence, and classifying them by conversation intent.

The first artificial neural network model 310 may include a KoBERT model. The KoBERT model may be a model trained with Korean sentence data collected from news and encyclopedias to overcome the limitations of the existing BERT's Korean performance. The KoBERT model shows higher performance than BERT in Korean natural language processing because it reflects characteristics of Korean well.

The training device may perform transfer learning of the first artificial neural network model 310 using pytorch (1.13.1 ver) and transformers (3.0.2 ver). At this time, training parameters are used identically except for two parameters num_epochs and log_interval used in a Naver review classifications example released by SKT Brain, wherein num_epochs may be adjusted from 5 to 15 and log_interval from 200 to 100.

The training device according to an embodiment may input depressive disorder-related expression data into the first artificial neural network model 310, label the depressive disorder-related expression data according to depressive disorder diagnosis criteria, and generate labeled depressive disorder-related expression data.

The depressive disorder-related expression data may include Everytime crawling data. The Every time crawling data may be data crawled from Everytime posts of a specific university. The Everytime is a university community that supports 400 universities nationwide, and is an online community where users can communicate anonymously with people from their university. The Everytime is the most familiar community to college students, and because anonymity is guaranteed, users can freely share their thoughts and feelings. In addition, the Every time has various types of bulletin boards, so it is easy for users to communicate with people of similar types.

To compare posts containing depressive emotions with daily posts, Everytime crawling data consisting of three data sets may be used. First, to collect posts containing depressive emotions (hereinafter, depression posts), all posts from the Everytime's "Mental Health bulletin board" and "Depression bulletin board" are extracted and utilized. To collect daily posts, posts (hereinafter, daily posts) from the "Humanities and Social Sciences campus bulletin board," "Natural Sciences campus bulletin board," "Seoul campus bulletin board," and "Global campus bulletin board" are utilized. The text and posting dates of Everytime posts are crawled using a selenium library. In addition, considering that the number of depression posts is insufficient compared to daily posts, the Every time crawling data may be constructed based on posts related to depressive emotions (hereinafter, daily-depression posts) from among the posts found when searching for the word "depressed" on the daily bulletin board.

The training device may tokenize the Everytime crawling data into sentence units. For tokenization, the training device may use NLTK, KSS (Korean sentence segmentation), a Korean sentence segmentation module, and Kiwi (Korean intelligent word identifier), a Korean morphological analyzer, and finally use Kiwi tokenized with the largest number of sentences. In addition, the training device may remove unnecessary sentences that are not related to depression, such as URL links or song lyrics, from segmented sentences.

The training device predicts labels for depression by inputting tokenized Every time data into sentence units into the first artificial neural network model 310 (e.g., the KoBERT model) generated through transfer learning. The training device classifies each sentence into nine depressive disorder diagnostic criteria according to the DSM-5 and performs labeling according to keywords corresponding to each classification. For example, example keywords for 'depressed mood' include 'tears', 'depressed', 'lonely', etc., and example keywords for 'anxiety and delay' include 'nervous', 'annoyed', 'scary', etc. The nine classification criteria may be labeled in order with numbers from 0 to 8.

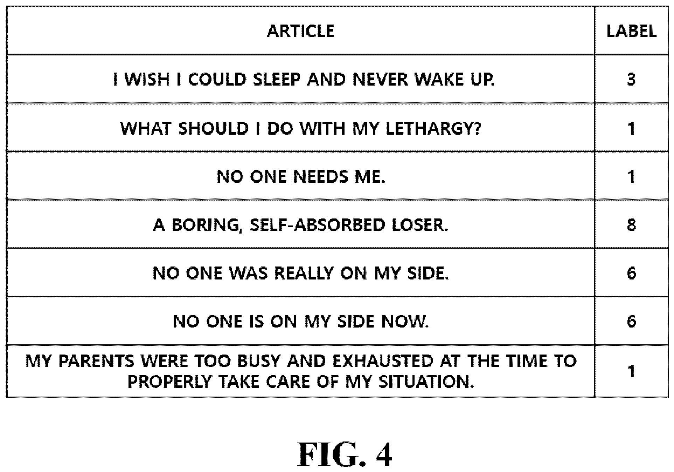

FIG. 4 is a view illustrating an example of a labeling result according to an embodiment.

Referring to FIG. 4, the word containing 'lethargy' corresponds to 'decreased interest or pleasure' (1) from among the nine categories. This is appropriately labeled as 1. The expression 'no one is on my side' is not included in a labeling dictionary, but it can be seen that it was appropriately predicted as 'feelings of worthlessness and guilt.'

However, in the case of depressive emotions, it may not be appropriate to express them with a single label due to their nature. Accordingly, the training device may perform multi-labeling using the first artificial neural network model 310.

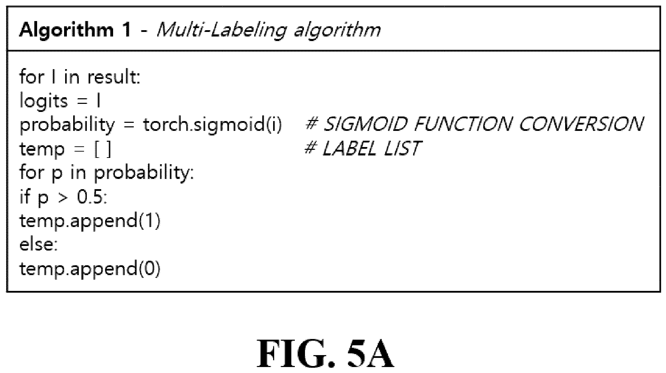

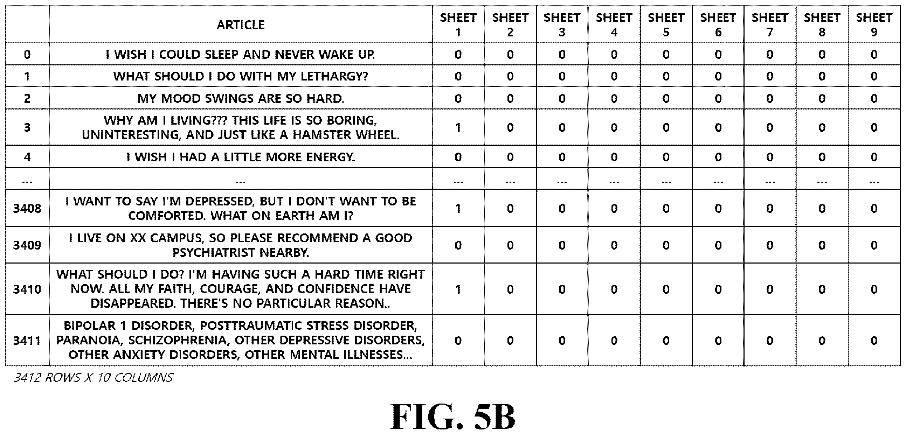

FIGS. 5A and 5B are views for explaining multi-labeling.

Referring to FIG. 5A, a training device according to an embodiment may convert a result value into a value between 0 and 1 using a sigmoid function, and may label a label having a value equal to or greater than 0.5 as the result value.

In more detail, the training device according to an embodiment may obtain first output data by inputting depressive disorder-related expression data into a first artificial neural network model, and may convert the first output data into second output data based on the sigmoid function.

The second output data may include a plurality of components corresponding to depressive disorder diagnostic criteria, respectively. For example, when using the DSM-5 depressive disorder diagnostic criteria, the second output data may include nine components.

The training device may label depressive disorder-related expression data based on depressive disorder diagnostic criteria corresponding to a component equal to or greater than a preset threshold value from among the plurality of components.

Referring to FIG. 5B, the depressive disorder-related expression data may be labeled with a plurality of diagnostic criteria.

However, when a sentence is tokenized into words and classified only by whether they contain keywords based on the DSM-5, expressions such as 'hopeless' may not be labeled. Therefore, the training device may additionally check for identical expressions and finally aggregate the results. In more detail, the training device may perform data augmentation on residual data from among the depressive disorder-related expression data that is not labeled by the first artificial neural network model, and re-input the residual data expanded by the data augmentation into the first artificial neural network to second-label the expanded residual data according to the depressive disorder diagnosis criteria.

Referring to FIG. 3 again, the training device may train a second artificial neural network model 320 based on the labeled depressive disorder-related expression data so that the second artificial neural network model 320 may output depressive disorder diagnosis criteria corresponding to input data. The training device may train the second artificial neural network model 320 so that the second artificial neural network model 320 may output not only depressive disorder diagnosis criteria corresponding to input data but also the probability corresponding to the depressive disorder diagnosis criteria.

The input data may be a new sentence that is the target of inference of the trained second artificial neural network model 320. The second artificial neural network model 320 may include a GRU model. The GRU model may be an improved model of RNN, which has strengths in sequence data processing and is highly utilized in the field of natural language processing. The GRU model may be a model that reduces and simplifies complex calculations while maintaining a solution to the long-term dependency problem of LSTM, which is similar to GRU. The training device may use the GRU model with a small number of parameters because the amount of data is relatively small.

When input data "I feel depressed, suffocated, and lethargic, so I can't sleep" is input to the trained second artificial neural network model 320, the probability of corresponding to a depressed mood (Sheet1) is 99 %, and the probability of corresponding to a decrease in interest or pleasure (Sheet2) is 76 %.

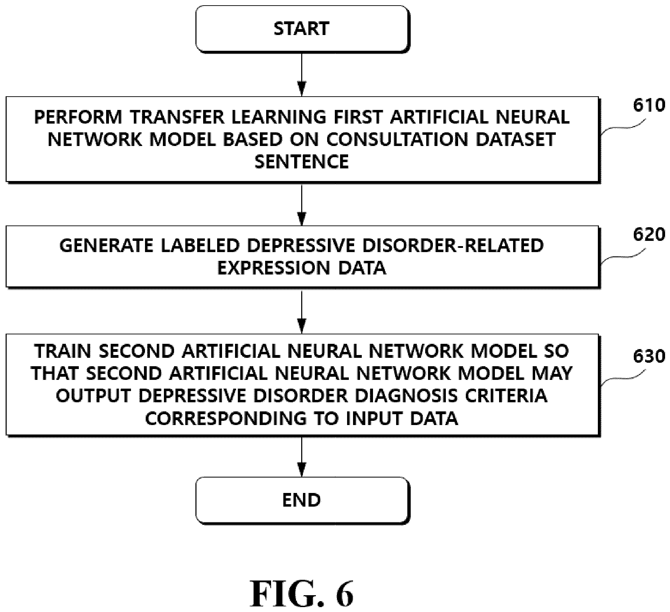

FIG. 6 is a flowchart for explaining a training method according to an embodiment.

Referring to FIG. 6, operations 610 to 630 are described as being performed by the training device 10 described with reference to FIG. 1. However, these operations 610 to 630 may be used via any other suitable electronic device and within any suitable system.

In addition, operations of FIG. 6 may be performed in the illustrated order and manner, but the order of some operations may be changed or some operations may be omitted without departing from the spirit and scope of the illustrated embodiment. A number of operations shown in FIG. 6 may be performed in parallel or concurrently.

In operation 610, a training device according to an embodiment may perform transfer learning a first artificial neural network model based on a consultation dataset sentence.

In operation 620, the training device according to an embodiment may input depressive disorder-related expression data into the first artificial neural network model, label the depressive disorder-related expression data according to depressive disorder diagnosis criteria, and generate labeled depressive disorder-related expression data.

The training device according to an embodiment may extract a plurality of depressive disorder diagnostic criteria corresponding to the depressive disorder-related expression data from depressive disorder diagnostic criteria, and multi-label the depressive disorder-related expression data based on the plurality of depressive disorder diagnostic criteria.

The training device according to an embodiment may obtain first output data by inputting the depressive disorder-related expression data into the first artificial neural network model, and may convert the first output data into second output data based on a sigmoid function. The second output data may include a plurality of components corresponding to the depressive disorder diagnostic criteria, respectively. The training device may label the depressive disorder-related expression data based on depressive disorder diagnostic criteria corresponding to a component equal to or greater than a preset threshold value from among the plurality of components.

The training device according to an embodiment may perform data augmentation on residual data from among the depressive disorder-related expression data that is not labeled by the first artificial neural network model, and re-input the residual data expanded by the data augmentation into the first artificial neural network to label the expanded residual data according to the depressive disorder diagnosis criteria.

In operation 630, the training device according to an embodiment may train a second artificial neural network model based on the labeled depressive disorder-related expression data so that the second artificial neural network model may output depressive disorder diagnosis criteria corresponding to input data.

The training device according to an embodiment may train the second artificial neural network model 320 so that the second artificial neural network model 320 may output depressive disorder diagnosis criteria corresponding to input data and the probability corresponding to the depressive disorder diagnosis criteria.

The embodiments described above may be implemented by hardware components, software components, and/or any combination thereof. For example, the devices, the methods, and components described in the embodiments may be implemented by using general-purpose computers or special-purpose computers, such as a processor, a controller, an arithmetic logic unit (ALU), a digital signal processor, a microcomputer, a field programmable gate array (FPGA), a programmable logic unit (PLU), a microprocessor, or any other devices which may execute and respond to instructions. A processing apparatus may execute an operating system (OS) and a software application executed in the OS. Also, the processing apparatus may access, store, operate, process, and generate data in response to the execution of software. For convenience of understanding, it may be described that one processing apparatus is used. However, one of ordinary skill in the art will understand that the processing apparatus may include a plurality of processing elements and/or various types of processing elements. For example, the processing apparatus may include a plurality of processors or a processor and a controller. Also, other processing configurations, such as a parallel processor, are also possible.

The software may include computer programs, code, instructions, or any combination thereof, and may construct the processing apparatus for desired operations or may independently or collectively command the processing apparatus. In order to be interpreted by the processing apparatus or to provide commands or data to the processing apparatus, the software and/or data may be permanently or temporarily embodied in any types of machines, components, physical devices, virtual equipment, computer storage mediums, or transmitted signal waves. The software may be distributed over network coupled computer systems so that it may be stored and executed in a distributed fashion. The software and/or data may be recorded in a computer-readable recording medium.

A method according to an embodiment may be implemented as program instructions that can be executed by various computer devices, and recorded on a computer-readable recording medium. The computer-readable recording medium may include program instructions, data files, data structures or a combination thereof. Program instructions recorded on the medium may be particularly designed and structured for embodiments or available to one of ordinary skill in a field of computer software. Examples of the computer-readable recording medium include magnetic media, such as a hard disc, a floppy disc, and magnetic tape; optical media, such as a compact disc-read only memory (CD-ROM) and a digital versatile disc (DVD); magneto-optical media, such as floptical discs; and hardware devices specially configured to store and execute program instructions, such as ROM, random-access memory (RAM), a flash memory, etc. Program instructions may include, for example, high-level language code that can be executed by a computer using an interpreter, as well as machine language code made by a complier.

In concluding the detailed description, those skilled in the art will appreciate that many variations and modifications may be made to the preferred embodiments without substantially departing from the principles of the present invention. Therefore, the disclosed preferred embodiments of the invention are used in a generic and descriptive sense only and not for purposes of limitation.

## Claims

1. A training method comprising:
performing transfer learning a first artificial neural network model based on a consultation dataset sentence;
inputting depressive disorder-related expression data into the first artificial neural network model, labeling the depressive disorder-related expression data according to depressive disorder diagnosis criteria, and generating labeled depressive disorder-related expression data; and
training a second artificial neural network model based on the labeled depressive disorder-related expression data so that a second artificial neural network model may output the depressive disorder diagnosis criteria corresponding to input data.

2. The training method of claim 1, wherein the generating of labeled depressive disorder-related expression data comprises:
extracting a plurality of depressive disorder diagnostic criteria corresponding to the depressive disorder-related expression data from the depressive disorder diagnostic criteria; and
multi-labeling the depressive disorder-related expression data based on the plurality of depressive disorder diagnostic criteria.

3. The training method of claim 1, wherein the generating of labeled depressive disorder-related expression data comprises:
obtaining first output data by inputting the depressive disorder-related expression data into the first artificial neural network model;
converting the first output data into second output data based on a sigmoid function, the second output data comprising a plurality of components corresponding to the depressive disorder diagnostic criteria, respectively; and
labeling the depressive disorder-related expression data based on the depressive disorder diagnostic criteria corresponding to a component equal to or greater than a preset threshold value from among the plurality of components.

4. The training method of claim 1, wherein the depressive disorder diagnostic criteria comprise DMS-5 depressive disorder diagnostic criteria.

5. The training method of claim 1, wherein the training comprises:
training the second artificial neural network model so that the second artificial neural network model may output the depressive disorder diagnosis criteria corresponding to the input data and probability corresponding to the depressive disorder diagnosis criteria.

6. The training method of claim 1, wherein the generating of labeled depressive disorder-related expression data comprises:
performing data augmentation on residual data from among the depressive disorder-related expression data that is not labeled by the first artificial neural network model; and
re-inputting residual data expanded by the data augmentation into the first artificial neural network to label the expanded residual data according to the depressive disorder diagnosis criteria.

7. The training method of claim 1, wherein the first artificial neural network model comprises a KoBERT model, and
the second artificial neural network model comprises a GRU model.

8. A computer program stored on a medium for executing the method of any one of claims 1 to 7 in combination with hardware.

9. A training device comprising:
a first artificial neural network model that performs transfer learning on sentences from a consultation dataset, labels depressive disorder-related expression data according to depressive disorder diagnosis criteria, and generates labeled depressive disorder-related expression data; and
a second artificial neural network model trained to output the depressive disorder diagnosis criteria corresponding to the labeled depressive disorder-related expression data.

10. The training device of claim 9, wherein the first artificial neural network model is configured to:
extract a plurality of depressive disorder diagnostic criteria corresponding to the depressive disorder-related expression data from the depressive disorder diagnostic criteria; and
multi-label the depressive disorder-related expression data based on the plurality of depressive disorder diagnosis criteria to generate the labeled depressive disorder-related expression data.

11. The training device of claim 9, wherein the first artificial neural network model is configured to:
obtain first output data by inputting depressive disorder-related expression data into the first artificial neural network model;
convert the first output data into second output data including a plurality of components corresponding to the depressive disorder diagnosis criteria, respectively, based on a sigmoid function; and
label the depressive disorder-related expression data based on the depressive disorder diagnostic criteria corresponding to a component equal to or greater than a preset threshold value from among the plurality of components to generate the labeled depressive disorder-related expression data.

12. The training device of claim 9, wherein the depressive disorder diagnostic criteria comprise DMS-5 depressive disorder diagnostic criteria.

13. The training device of claim 9, wherein the second artificial neural network model is trained to output the depressive disorder diagnosis criteria corresponding to the labeled depressive disorder-related expression data and probability corresponding to the depressive disorder diagnosis criteria.

14. The training device of claim 9, wherein the first artificial neural network model is configured to:
perform data augmentation on unlabeled residual data from among the depressive disorder-related expression data; and
re-input expanded residual data through the data augmentation, label the expanded residual data according to the depressive disorder diagnosis criteria, and generate the labeled depressive disorder-related expression data.

15. The training device of claim 9, wherein the first artificial neural network model comprises a KoBERT model, and
the second artificial neural network model comprises a GRU model.
